# FASCICULE DE BREVET EUROPEEN

(11) **EP 2 068 756 B1**
(45) Date de publication et mention de la délivrance du brevet: **05.11.2014**
(21) Numéro de dépôt: 07823375.6
(22) Date de dépôt: 31.07.2007
(51) Int. Cl.: A61F 2/00

(54) **HAMAC SOUS-URETRAL**
SUBURETHRALE HÄNGEMATTE
SUBURETHRAL HAMMOCK

(30) Priorité: 02.08.2006 US 821153 P
(43) Date de publication de la demande: 17.06.2009
(73) Titulaire: Cousin Biotech, S.A.S., 59117 Werwicq Sud (FR)
(72) Inventeur: Dubernard, Pierre, 69006 Lyon (FR)
(74) Mandataire: Balesta, Pierre
(86) Numéro de dépôt international: PCT/FR2007/001321
(87) Numéro de publication internationale: WO 2008/015338

(56) Documents cités:
- WO-A-01/45589
- WO-A-02/30293
- WO-A-2005/110274
- US-A- 5 527 276
- US-A1- 2004 015 048
- US-A1- 2004 087 970
- US-B1- 6 221 005

## Description

### DOMAINE TECHNIQUE

La présente invention concerne le domaine général des dispositifs médicaux destinés au traitement de l'incontinence, notamment urinaire.

La présente invention concerne plus particulièrement une prothèse implantable destinée au traitement de l'incontinence comprenant un moyen de soutènement présentant un organe d'appui conçu pour venir se placer sous l'urètre afin de faire obstacle à l'éloignement de ce dernier en direction de la partie inférieure du pelvis et d'empêcher tout écoulement urinaire intra-urétral incontrôlé, telle que revendiquée dans la revendication 1.

### TECHNIQUE ANTERIEURE

Il est connu de recourir à des dispositifs médicaux implantés par voie chirurgicale afin de traiter les problèmes d'incontinence, et notamment d'incontinence urinaire.

Une telle incontinence peut notamment résulter d'une altération des fonctions sphinctériennes naturelles du patient, par exemple, chez l'homme, suite à une prostatectomie. Chez la femme, une autre forme d'incontinence dite « *d'effort* » peut également provenir d'une laxité des tissus pelviens créant une hyper-mobilité de l'urètre.

La nature du dispositif médical implanté dépend notamment de l'origine et de la sévérité de l'incontinence, ainsi que du sexe du patient.

Ainsi, en cas d'incontinence faible à modérée chez la femme, de type incontinence d'effort, il est connu d'implanter au patient une *« bandelette TVT »* positionnée transversalement sous l'urètre, dans les tissus, sensiblement sans tension.

Il est également connu d'implanter chez l'homme souffrant d'incontinence faible à modérée une plaque textile engagée sous l'urètre et tendue par fixation de chacune de ses extrémités à l'une des branches du rameau ischio-pubien.

En cas d'incontinence plus sévère, il est généralement nécessaire de recourir à un sphincter artificiel, qui comprend de façon classique une manchette destinée à entourer l'urètre et comportant sur sa face intérieure une poche annulaire gonflable dont le gonflage provoque l'occlusion de l'urètre par constriction de celui-ci.

Bien qu'ils procurent des résultats satisfaisants sur le plan thérapeutique, de tels dispositifs de l'art antérieur présentent toutefois des inconvénients non négligeables.

En effet, on constate parfois, après implantation d'une bandelette de type TVT, que son efficacité est insuffisante pour remédier au problème d'incontinence.

Il est alors nécessaire de procéder à une seconde intervention chirurgicale pour remplacer ladite bandelette par un sphincter artificiel, ce qui expose le patient à un nouveau traumatisme et à de nouvelles complications.

Par ailleurs, les sphincters artificiels connus sont particulièrement onéreux, et leur mise en place relève d'une intervention chirurgicale relativement complexe.

Les plaques textiles sont quant à elles fréquemment fixées dans l'os pubien au moyen d'organes d'ancrage, tels que des vis, qui augmentent le traumatisme subi par le patient et peuvent être à l'origine de complications. Le document US 622 1005 décrit une prothèse comprenant deux bretelles de suspension. Chaque bretelle ne comporte qu'une seule suspente reliant un organe d'appui à un organe porteur chevauchant l'os pubien.

### EXPOSE DE L'INVENTION

Les objets assignés à l'invention visent par conséquent à proposer une nouvelle prothèse implantable destinée au traitement de l'incontinence qui ne présente pas les inconvénients énumérés ci-dessus et qui permette de traiter efficacement divers degrés d'incontinence tout en étant particulièrement simple à implanter.

Un autre objet assigné à l'invention vise à proposer une nouvelle prothèse qui soit particulièrement atraumatique et qui réduise sensiblement la gêne occasionnée au patient ainsi que le risque de complication postopératoire.

Un autre objet de l'invention vise à proposer une nouvelle prothèse qui soit de conception particulièrement simple et peu coûteuse.

Un autre objet assigné à l'invention vise à proposer une nouvelle prothèse qui présente une bonne stabilité mécanique et une bonne tenue dans le temps.

Un autre objet assigné à l'invention vise à proposer une nouvelle prothèse particulièrement adaptée au traitement de l'incontinence urinaire masculine.

Un autre objet assigné à l'invention vise à proposer une nouvelle prothèse dont la mise en oeuvre puisse être adaptée en fonction de l'anatomie du patient.

La présente invention vise également à proposer une nouvelle prothèse qui permette un traitement conjoint de l'incontinence urinaire féminine et d'une cervicocystoptose.

Les objets assignés à l'invention sont atteints à l'aide d'une prothèse implantable destinée au traitement de l'incontinence comprenant un moyen de soutènement présentant un organe d'appui conçu pour venir se placer sous l'urètre afin de faire obstacle à l'éloignement de ce dernier en direction de la partie inférieure du pelvis et d'empêcher tout écoulement intra-urétral incontrôlé, ladite prothèse comportant.
- un moyen de suspension atraumatique pourvu d'au moins deux bretelles de suspension comprenant chacune un organe porteur agencé pour chevaucher une partie de l'os pubien et prendre appui sur ce dernier, ainsi qu'au moins une suspente antérieure et une suspente postério agencées pour relier l'organe porteur au moyen de soutènement en passant respectivement devant et derrière l'os pubien, lesdites bretelles étant conçue pour maintenir l'organe d'appui à une distance sensiblement constante de l'os pubien, dite distance de soutènement,
- un moyen de réglage réversible de la distance de soutènement permettant de faire varier de manière réversible ladite distance de soutènement.

Il est aussi décrit un instrument chirurgical destiné à l'implantation d'une prothèse de traitement de l'incontinence comportant un moyen de soutènement présentant un organe d'appui, un moyen de suspension atraumatique pourvu d'un organe porteur agencé pour chevaucher une partie de l'os pubien et prendre appui sur ce dernier ainsi que d'au moins une suspente reliant l'organe porteur au moyen de soutènement afin de maintenir l'organe d'appui à une distance de soutènement sensiblement constante de l'os pubien, ledit instrument chirurgical étant pourvu d'un organe d'introduction conçu pour être enfoncé dans les tissus situés dans l'espace rétro-pubien afin d'y ouvrir un passage destiné à l'une ou l'autre des suspentes, ledit organe d'introduction comprenant au moins une portion proximale agencée pour pénétrer dans les tissus, une portion distale rattachée à un moyen de préhension, et un tronçon intermédiaire qui relie la portion proximale à la portion distale et présente une surface latérale extérieure au contact des tissus traversés, ledit instrument chirurgical étant caractérisé en ce que le tronçon intermédiaire comporte au moins un orifice latéral reliant sa surface latérale extérieure à un canal de collecte de sorte à pouvoir recueillir le liquide contenu dans la vessie lorsque l'organe d'introduction perfore accidentellement cette dernière.

Une méthode chirurgicale de traitement de l'incontinence est décrite comprenant une étape (a) d'engagement au cours de laquelle on engage sous l'urètre un moyen de soutènement comprenant un organe d'appui conçu pour faire obstacle à l'éloignement de l'urètre en direction de la partie inférieure du pelvis et empêcher tout écoulement intra-urétral incontrôlé, ladite méthode chirurgicale étant caractérisée en ce qu'elle comprend une étape (b) de suspension osseuse atraumatique au cours de laquelle on suspend le moyen de soutènement à l'os pubien à l'aide d'un moyen de suspension atraumatique de telle sorte que l'organe d'appui soit maintenu à une distance sensiblement constante de l'os pubien, et plus particulièrement de la symphyse pubienne, dite distance de soutènement d₀, ainsi qu'une étape (c) de réglage de tension de suspension au cours de laquelle le praticien ajuste *in vivo* la distance de soutènement afin de régler l'effort de suspension exercé par l'organe d'appui sur l'urètre.

### DESCRIPTIF SOMMAIRE DES DESSINS

D'autres objets, caractéristiques et avantages de l'invention apparaîtront plus en détails à la lecture de la description qui suit, ainsi qu'à l'aide des dessins annexés, fournis à titre purement illustratif et non limitatif, parmi lesquels :
- La figure 1 illustre, selon une vue de face antérieure, la mise en oeuvre d'une variante de prothèse implantable conforme à l'invention.
- La figure 2 illustre, selon une coupe schématique de côté, la mise en oeuvre chez l'homme d'une variante de prothèse conforme à l'invention.
- La figure 3 illustre, selon une vue schématique partielle en perspective, la mise en oeuvre d'une variante de réalisation de prothèse correspondant à celle de la figure 2.
- La figure 4 illustre, selon une vue partielle en coupe de face, le principe de fonctionnement chez l'homme d'une variante de prothèse conforme à l'invention.
- La figure 5 illustre, selon une vue de dessous, une variante de prothèse conforme à l'invention particulièrement adaptée au traitement de l'incontinence masculine.
- Les figures 6A et 6B illustrent, respectivement selon une vue partielle en perspective et une vue en coupe, une première variante de réalisation de moyen de réglage de distance de soutènement susceptible d'être mis en oeuvre au sein d'une prothèse implantable conforme à l'invention.
- La figure 7 illustre, selon une vue partielle en perspective, une seconde variante de réalisation de moyen de réglage de distance de soutènement susceptible d'être mis en oeuvre au sein d'une prothèse implantable conforme à l'invention.
- Les figures 8A et 8B illustrent, selon une vue partielle en perspective et une vue en coupe longitudinale, une troisième variante de réalisation de moyen de réglage de distance de soutènement susceptible d'être mis en oeuvre au sein d'une prothèse implantable conforme à l'invention.
- La figure 9 illustre, selon une vue schématique de dessus, une variante de réalisation de prothèse conforme à l'invention particulièrement adaptée au traitement conjoint de l'incontinence féminine et d'une cervicocystoptose.
- La figure 10 illustre, selon une vue en coupe de profil simplifiée, la mise en oeuvre chez la femme d'une variante de réalisation de prothèse conforme à l'invention.
- La figure 11 illustre, selon une vue coupe de profil simplifiée, la mise en oeuvre chez la femme de la prothèse représentée sur la figure 9.
- La figure 12 illustre, selon une vue partielle en perspective, une quatrième variante de réalisation de moyen de réglage de distance de soutènement susceptible d'être mis en oeuvre au sein d'une prothèse implantable conforme à l'invention.
- La figure 13 illustre, selon une vue d'ensemble en perspective, une variante de réalisation d'instrument chirurgical.
- La figure 14 illustre, selon une vue en coupe longitudinale partielle, une portion de l'instrument chirurgical de la figure 13.

### MEILLEURE MANIERE DE REALISER L'INVENTION

La présente invention concerne une prothèse 1 implantable destinée au traitement de l'incontinence, et plus préférentiellement de l'incontinence urinaire.

Avantageusement, la prothèse 1 conforme à l'invention peut être adaptée au traitement de divers types et degrés d'incontinence urinaire, chez l'homme ou chez la femme.

Ainsi, ladite prothèse 1 peut être adaptée au traitement, chez l'homme, de l'incontinence légère, modérée ou sévère provoquée par une insuffisance sphinctérienne, en suppléant la fonction sphinctérienne naturelle.

La prothèse 1 peut également être adaptée, chez la femme, au traitement de l'incontinence d'effort résultant d'une hyper-mobilité de l'urètre et du col de la vessie. En particulier, ladite prothèse 1 peut avantageusement être mise en oeuvre chez la femme lorsque d'autres techniques opératoires ont échoué.

La prothèse 1 conforme à l'invention comprend un moyen de soutènement 2 qui présente un organe d'appui 3, lequel est conçu pour venir se placer sous l'urètre U afin de faire obstacle à l'éloignement dudit urètre en direction de la partie inférieure du pelvis et d'empêcher tout écoulement intra-urétral incontrôlé.

Au sens de l'invention, l'organe d'appui 3 peut venir soit en contact direct avec la face inférieure de l'urètre, c'est-à-dire avec la portion de l'urètre sensiblement orientée vers les membres inférieurs du patient, soit en contact indirect sur des tissus sous-jacents accolés audit urètre.

La configuration de contact pourra notamment dépendre du sexe du patient. Ainsi, chez la femme, l'organe d'appui 3 sera de préférence appliqué directement contre l'urètre, tandis que chez l'homme, ledit organe d'appui 3 sera de préférence placé au contact du muscle bulbo-cavemeux MBC.

Bien entendu, l'état de surface et la superficie de la surface de l'organe d'appui 3 destinée à être effectivement plaquée contre les tissus (urètre ou muscle bulbo-caverneux) seront par ailleurs choisis de manière à conférer audit organe d'appui 3 un caractère sensiblement atraumatique.

Selon une caractéristique importante de l'invention, la prothèse 1 comporte un moyen de suspension atraumatique 4 pourvu d'au moins un organe porteur 5, ledit organe porteur étant agencé pour chevaucher une partie de l'os pubien OP et prendre appui sur ce dernier, ainsi que d'au moins une suspente 6, 7 reliant ledit organe porteur 5 au moyen de soutènement 2, ladite suspente 6, 7 étant conçue pour maintenir l'organe d'appui 3 à une distance sensiblement constante de l'os pubien OP, et plus particulièrement de la symphyse pubienne SP, dite distance de soutènement d₀.

En d'autres termes, le moyen de suspension atraumatique 4 est agencé pour lier mécaniquement le moyen de soutènement 2, et plus précisément son organe d'appui 3, à l'os pubien OP de telle sorte que ledit organe d'appui 3 fait obstacle à un éventuel débattement de l'urètre dans le sens de son éloignement de l'os pubien, et plus particulièrement de la symphyse pubienne SP. L'organe d'appui 3 forme ainsi une butée *« anti-chute* » qui retient sensiblement l'urètre à une distance (maximale) de soutènement d₀ prédéterminée de ladite symphyse pubienne en s'opposant sensiblement aux effets de la gravité.

Ainsi, l'organe d'appui 3 est susceptible d'éxercer un effort de retenue, ou *« effort de suspension »* F qui empêche l'urètre U de s'écarter de la symphyse pubienne, et plus précisément de s'affaisser vers le bas du pelvis.

Selon l'âge et la corpulence du patient, la distance de soutènement d₀ sera de préférence comprise entre environ 5 cm et 20 cm, et de préférence entre 8 cm et 15 cm.

Par « *suspension atraumatique* », on indique que le moyen de suspension 4, et plus particulièrement l'organe porteur 5, est conçu pour ne pas meurtrir les tissus sur lesquels il prend appui, et en particulier pour respecter l'intégrité de l'os pubien.

Plus particulièrement, le moyen de suspension 4 conforme à l'invention sera dépourvu de tout élément d'ancrage saillant coupant, perforant, ou abrasif, tels que des pointes, ergots, ou vis, susceptibles de perforer, lacérer, ou fendre l'os pubien, à sa surface comme en profondeur, et/ou les tissus voisins.

Selon une autre caractéristique importante de l'invention, la prothèse 1 comporte un moyen de réglage réversible 20 de la distance de soutènement d₀ permettant de faire varier de manière réversible ladite distance de soutènement d₀.

Par « *réversible* », on indique que le moyen de réglage autorise aussi bien l'augmentation que la réduction de la distance de soutènement d₀, ledit réglage étant cependant « *fixe »* au sens où la distance de soutènement d₀ est durablement maintenue une fois la prothèse implantée. Ainsi, le moyen de réglage 20 est conçu pour conserver sensiblement constante ladite distance de soutènement, en fonctionnement normal, de telle sorte que cette dernière n'est par exemple pas modifiable spontanément de manière dynamique sous la contrainte des divers organes du patient.

Toutefois, ladite distance de soutènement peut avantageusement être de nouveau modifiée sans difficulté au cours d'un acte thérapeutique volontaire du médecin, par exemple lors d'une intervention chirurgicale ultérieure mineure par laquelle le chirurgien accède au moyen de réglage par une simple et courte incision sus-pubienne, sans avoir à réaliser de dissection importante destinée à dégager complètement l'urètre.

Ledit moyen de réglage 20 permet avantageusement au praticien de choisir librement, notamment lors de l'implantation de la prothèse 1, la distance à laquelle il place la butée que forme l'organe d'appui 3, et d'ajuster la distance de soutènement en fonction de l'anatomie propre du patient. Un même modèle de prothèse 1 standard peut ainsi être utilisé pour traiter de nombreux patients.

En outre, de façon particulièrement avantageuse, le moyen de réglage 20 permet, en fonction de l'effet de serrage obtenu par le rapprochement forcé plus ou moins prononcé du moyen de soutènement 2 et de la symphyse pubienne SP, de moduler l'intensité de l'effort de suspension F.

Plus précisément, plus on cherche à réduire la distance d₀, plus on induit une traction dans les suspentes 6, 7 et plus on tend à comprimer l'urètre U contre les tissus qui le surplombent, en exerçant sur ledit urètre une contrainte dirigée de bas en haut et sensiblement orientée selon la direction d'une droite fictive tirée entre la base de l'urètre U qui se trouve en contact avec l'organe d'appui 3 et la symphyse pubienne SP.

Ainsi, il est possible d'opter pour une suspension « *active »* dans laquelle la prothèse 1 exerce en permanence sur l'urètre un effort de suspension F non nul. En particulier, une telle solution pourra être retenue pour remédier à l'incontinence urinaire chez l'homme.

A l'inverse, il est possible d'obtenir, avec le même moyen de réglage 20, une suspension *« passive »,* c'est-à-dire un maintien lâche, de l'urètre en réglant la distance de soutènement d₀ de telle sorte qu'elle corresponde sensiblement à la distance anatomique naturelle au repos et que l'effort de suspension F exercé par la prothèse 1 soit sensiblement nul par défaut.

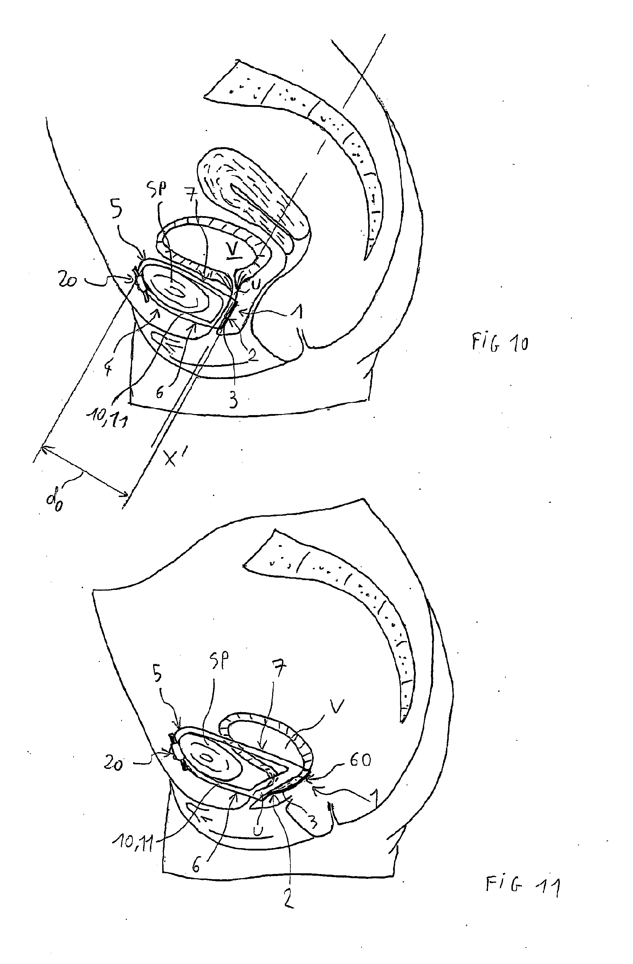

En d'autres termes, il est possible d'utiliser le moyen de réglage 20 pour positionner l'organe d'appui 3 sensiblement au contact de l'urètre, sans contrainte, ou *« sans tension » ,* tel que cela est illustré sur la figure 10, de telle sorte que ledit organe d'appui 3 forme une sorte de barrière et qu'une contrainte n'apparaisse que lorsque l'urètre tend à *« fuir »* la symphyse pubienne en tirant sur les suspentes. Une telle solution est particulièrement indiquée chez femme pour le traitement de l'incontinence d'effort, en raison notamment du risque érosion des tissus particulièrement fins de l'urètre.

Ainsi, l'organe d'appui 3 conforme à l'invention peut être plus ou moins compressif et sa mise en oeuvre adaptée selon que l'on cherche à simplement maintenir sans compression résiduelle le plancher urétral en configuration fonctionnelle (chez la femme), ou que l'on cherche à exercer et maintenir une compression résiduelle permanente de l'urètre (chez l'homme).

Selon une variante de réalisation (non représentée), le moyen de suspension 4 peut être agencé pour permettre la suspension *«* en *transobturateur »* du moyen de soutènement 2, c'est-à-dire que l'organe porteur 5 est conçu pour venir prendre appui sur une branche ischio-pubienne ou sur une branche ilio-pubienne, et plus particulièrement sur la partie supérieure de l'une ou l'autre desdites branches.

Selon une telle variante de réalisation, l'une ou l'autre des suspentes 6, 7 est de préférence agencée pour relier le moyen de soutènement 2 à l'organe porteur 5 en passant par le trou obturateur.

Toutefois, de façon particulièrement préférentielle, le moyen de suspension 4 constitue un moyen de suspension péri-symphysaire conçu pour suspendre le moyen de soutènement 2 à la symphyse pubienne SP.

Un tel mode de suspension présente en effet de nombreux avantages en ratière de stabilité, d'efficacité fonctionnelle et de simplicité de la suspension.

Par commodité de description, on considèrera donc dans la suite du texte que la prothèse 1 est destinée à être suspendue à la symphyse pubienne, sans que cela ne constitue une limitation de l'invention.

De préférence, le moyen de suspension 4 comprend au moins une suspente antérieure 6 et une suspente postérieure 7 agencées pour relier l'organe porteur 5 au moyen de soutènement 2 en passant respectivement devant et derrière la symphyse pubienne SP.

L'organe porteur 5 peut donc avantageusement venir se positionner autour de l'os pubien, de sorte à épouser au moins en partie le contour de ce dernier, et plus particulièrement la partie supérieure dudit contour.

Au sens de l'invention, l'organe porteur 5 forme un point d'appui mécanique, c'est-à-dire présente une surface de contact avec les tissus sous-jacents, surface au niveau de laquelle peut être transmis un effort, en l'occurrence permettant d'assurer la suspension.

A ce titre, il est remarquable que l'os pubien OP, et plus particulièrement la symphyse pubienne SP, offre un point de suspension particulièrement rigide, résistant et stable, qui confère une bonne assise à la prothèse 1.

De plus, la suspension conforme à l'invention est avantageusement une suspension « *libre* », c'est-à-dire que l'organe porteur 5 est simplement posé sur la symphyse pubienne, sans qu'il soit nécessaire de l'y arrimer par exemple par suture, agrafage ou nouage.

En outre, bien qu'il soit envisageable d'engager l'organe porteur 5 au-dessus de la-symphyse pubienne à distance de celle-ci, en comprimant ainsi les tissus emprisonnés entre ledit organe porteur 5 et la surface supérieure de la symphyse pubienne SP (tels que des aponévroses musculaires), il est toutefois préférable que l'organe porteur 5 vienne au contact de l'os pubien et repose directement sur le bord supérieur de la symphyse pubienne qu'il chevauche.

Avantageusement, une telle disposition garantit le maintien latéral de la prothèse 1, et plus précisément de l'organe porteur 5, par l'épine pubienne qui fait office de butée.

Selon une variante de réalisation non représentée, l'organe porteur 5 peut se présenter sous la forme d'une sorte de selle en U destinée à venir coiffer la symphyse pubienne.

Il est remarquable que le moyen de suspension 4 péri-symphysaire conforme à l'invention offre au moyen de soutènement 2 un point de fixation mécanique qui se situe sensiblement à l'aplomb de l'urètre U, et permet donc la réalisation d'une suspension « *directe* », c'est-à-dire qui, contrairement aux bandelettes ou aux plaques de l'art antérieur, fait sensiblement coïncider la direction de l'effort de suspension F avec la direction de sollicitation en traction des éléments constitutifs du moyen de suspension 4:

En effet, les bandelettes ou les plaques de l'art antérieur sont prévues pour être disposées transversalement sous le pubis, et sous l'urètre, de telle sorte que, pour générer un effort de suspension résultant, même faible, elles requièrent une mise en traction transversale importante qui peut traumatiser les tissus mous ou les tissus osseux dans lesquels sont ancrées leurs extrémités.

C'est pourquoi le moyen de suspension 4 préférentiel conforme à l'invention permet avantageusement de minimiser les efforts subis par les tissus de fixation, puisqu'il convertit directement la quasi-totalité des efforts exercés sur lesdits tissus de fixation en une résultante utile d'effort de suspension F.

En outre, tel que cela sera détaillé ci-après, un tel mode de fixation symphysaire permet avantageusement un maintien plus stable et plus enveloppant de l'urètre, améliore la capacité de continence urinaire de la prothèse 1 et s'avère plus simple à implanter qu'un sphincter artificiel.

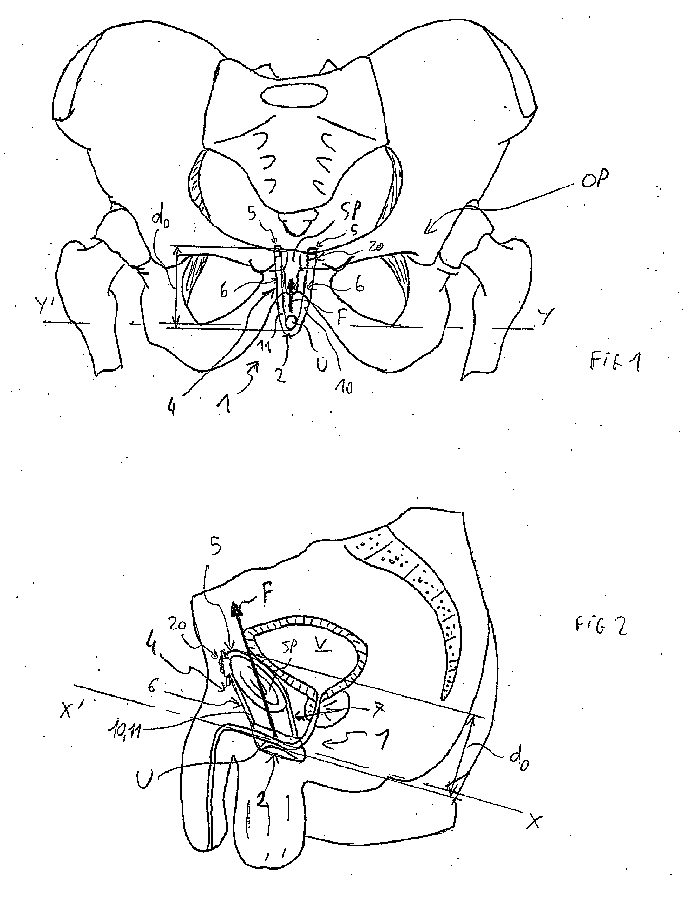

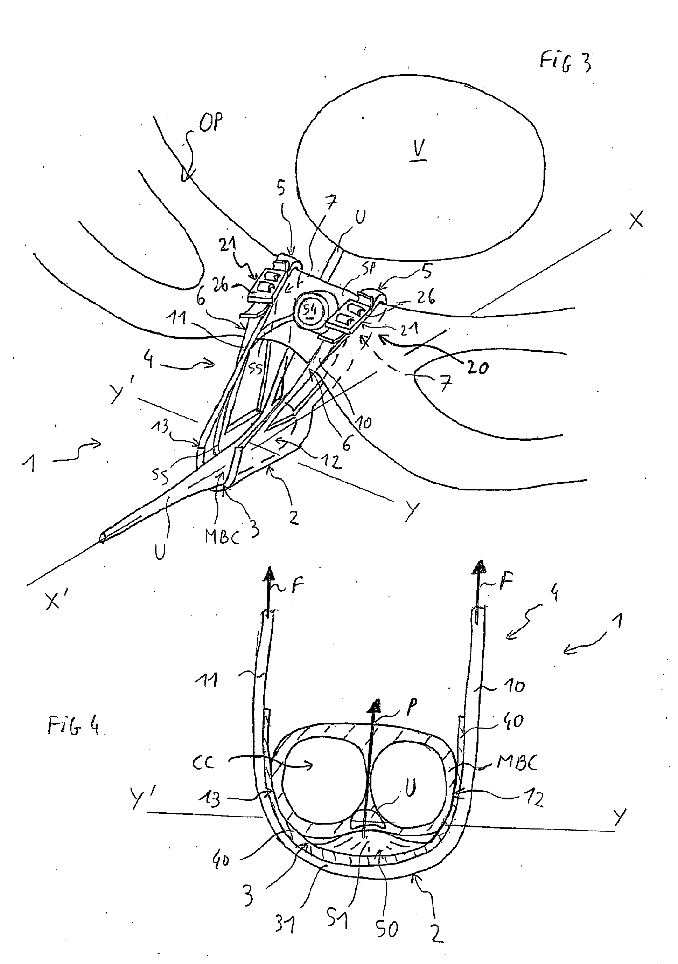

De préférence, tel que cela est illustré notamment sur les figures 1 et 3, le moyen de suspension 4 comporte au moins deux bretelles de suspension 10, 11 qui comprennent chacune une suspente antérieure 6, un organe porteur 5, et une suspente postérieure 7.

Bien entendu, les éléments susmentionnés peuvent être initialement distincts et assemblés par un moyen de jonction mécanique quelconque ou, a *contrario*, l'une et/ou l'autre des suspentes 6, 7 peuvent être venues de matière avec l'organe porteur 5 et/ou avec le moyen de soutènement 2.

Par convention, l'adjectif « *antérieur* » fait référence à un élément se trouvant dans l'espace compris entre la symphyse pubienne et les téguments antérieurs du patient, tandis que l'adjectif « *postérieur* » désigne un élément rétro-pubien situé entre la symphyse pubienne et l'excavation pelvienne.

Ainsi, tel que cela est illustré sur les figures 1 à 3 et 7, chaque bretelle de suspension 10, 11 forme sensiblement une arche dont les suspentes forment les pieds et dont la portion courbe, qui vient envelopper, ou « *enfourcher* », la partie supérieure de la symphyse pubienne, forme l'organe porteur 5.

Tel que cela est illustré notamment sur les figures 2, 3 ou 10, chaque bretelle forme ainsi avantageusement, en coopération avec une partie du moyen de soutènement 2, une boucle, de préférence simple, dans laquelle passe la symphyse pubienne.

De préférence, l'organe d'appui 3, et plus globalement le moyen de soutènement 2, est divisé en une zone gauche 3G et une zone droite 3D situées de part et d'autre d'une ligne moyenne (L) fictive qui se trouve sensiblement parallèle à la direction principale d'extension (XX') de l'urètre U lorsque la prothèse est implantée, et le moyen de suspension 4 possède une bretelle gauche 10 et une bretelle droite 11, lesdites bretelles gauche et droite 10, 11 étant respectivement fixées au moyen de soutènement 2 dans la zone gauche 3G et dans la zone droite 3D.

Un tel agencement permet avantageusement d'équilibrer et de stabiliser la suspension en permettant au praticien de faire passer la bretelle gauche à gauche de l'urètre et la bretelle droite à droite de l'urètre.

Par ailleurs, le moyen de soutènement 2 comporte de préférence, tel que cela est illustré sur les figures 3 et 4, des organes de maintien latéral 12, 13, en l'occurrence au moins un organe de maintien latéral gauche 12 et un organe de maintien latéral droit 13, conçus pour venir se positionner de chaque côté de l'urètre U et faire obstacle au débattement latéral de ce ernier.

De façon particulièrement préférentielle, les organes de maintien latéral 12, 13 sont venus de matière avec l'organe d'appui 3.

Une telle variante de réalisation permet avantageusement d'une part d'envelopper sensiblement l'urètre dans une structure porteuse formant une sorte de hamac, ou de harnais de contention, limitant intrinsèquement le débattement de l'urètre dans les directions latérale gauche, latérale droite et verticale inférieure, et d'autre part de stabiliser le moyen de soutènement 2 à l'aide de deux structures triangulaires formées par les suspentes de chacune des bretelles 10, 11.

Plus globalement, il est remarquable que la prothèse 1 conforme à l'invention présente de préférence une structure globalement symétrique, c'est-à-dire que sa portion gauche correspond sensiblement à l'image de sa portion droite par un plan de symétrie qui correspond sensiblement au plan sagittal du patient lui-même.

L'homme du métier sera naturellement en mesure d'apprécier la longueur idoine des bretelles 10, 11 en fonction de l'âge et de la corpulence du patient.

A titre indicatif, la longueur totale de chacune desdites bretelles 10, 11 sera de préférence comprise entre 10 cm et 50 cm environ, et de façon encore plus préférentielle entre 15 cm et 25 cm environ.

De préférence, les bretelles, et plus généralement le moyen de suspension 4, sont conçus pour être suffisamment flexibles pour permettre leur implantation, et notamment leur inflexion au-dessus de la symphyse, tout en étant peu extensibles de manière à pouvoir supporter sensiblement sans déformation longitudinale l'effort de traction assurant la suspension du moyen de soutènement 2 à ladite symphyse.

En outre, lesdites bretelles seront de préférence non adhérentes.

Ainsi, l'une et/ou l'autre des bretelles 10, 11 pourra être formée à base d'une bande en matériau polymère, par exemple en poly-tétra-fluor-éthylène (PTFE).

L'une et/ou l'autre des bretelles 10, 11 pourra également être formée à base d'une bande d'un matériau composite comprenant un insert de textile implantable, de type PolyPropylène ou PolyEster, et une matrice ou un revêtement en élastomère de type silicone:

Il est également envisageable d'effectuer un traitement de surface en vue de rendre le textile anti-adhérent, par exemple par application d'un procédé plasma de gaz fluoré de type C₂H₂F₄.

De préférence, ladite bande aura une largeur sensiblement comprise entre 0,5 cm et 3 cm, et de façon particulièrement préférentielle sensiblement égale à 1 cm, tandis que son épaisseur sera de préférence comprise entre 0,2 mm et 1 mm.

Avantageusement, l'utilisation de bandes permet aux plats des bretelles de venir se positionner sensiblement autour de l'os pubien, en épousant la forme de ce dernier de manière à répartir les contraintes de suspension sur une surface relativement importante.

Selon une autre variante de réalisation (non représentée), en particulier lorsque la suspension se fait quasiment sans tension, par exemple chez la femme, les bretelles pourront être formées par des cordelettes, par exemple de diamètre compris entre 1 mm et 3 mm.

En outre, afin de faciliter l'implantation de la prothèse 1, l'une et/ou l'autre des bretelles est, avant implantation, divisée en un premier brin 10A, 11A et un second brin 10B, 11B qui présentent chacun une extrémité captive, fixée au moyen de soutènement 4, et une extrémité libre.

Ainsi, avantageusement, chaque bretelle 10, 11 peut être reconstituée *in vivo* - par la réunion de l'extrémité libre de son premier brin 10A, 11A avec l'extrémité libre de son second brin 10B, 11 B, lesdits premier et second brins ayant préalablement été enfilés dans les tissus de part et d'autre de la symphyse pubienne.

En particulier, les premier et second brins peuvent sensiblement correspondre aux suspentes antérieure et postérieure respectivement.

Par ailleurs, selon une variante de réalisation non représentée, il est tout à fait envisageable que l'une ou l'autre des bretelles soit formée par un brin unique attaché par l'une de ses extrémités au moyen de soutènement 2 et dont l'extrémité libre peut être rabattue sur ledit moyen de soutènement 2 après avoir été préalablement passée autour de la symphyse.

D'autre part, selon une variante d'agencement préférentielle (non représentée), le moyen de réglage 20 de la distance de soutènement sera disposé/sensiblement à proximité du moyen de soutènement 2, à la base de la suspente antérieure 6, afin d'être facilement accessible et manoeuvrable par le chirurgien.

De préférence, tel que cela est notamment illustré sur la figure 3, le moyen de réglage 20 de la distance de soutènement possède un organe d'ajustement de longueur 21 de l'une ou l'autre des suspentes 6, 7, et plus précisément de l'une ou l'autre des bretelles 10,11.

Plus particulièrement, le moyen de réglage réversible de la distance de soutènement d₀ autorise aussi bien l'allongement que le raccourcissement desdites bretelles 10,11.

Bien entendu, tout moyen de réglage discret ou continu de la longueur des bretelles entre dans le cadre de l'invention.

Ainsi, selon une variante de réalisation, le moyen de réglage réversible 20 de la distance de soutènement est conçu pour permettre une modification discrète de la distance de soutènement d₀.

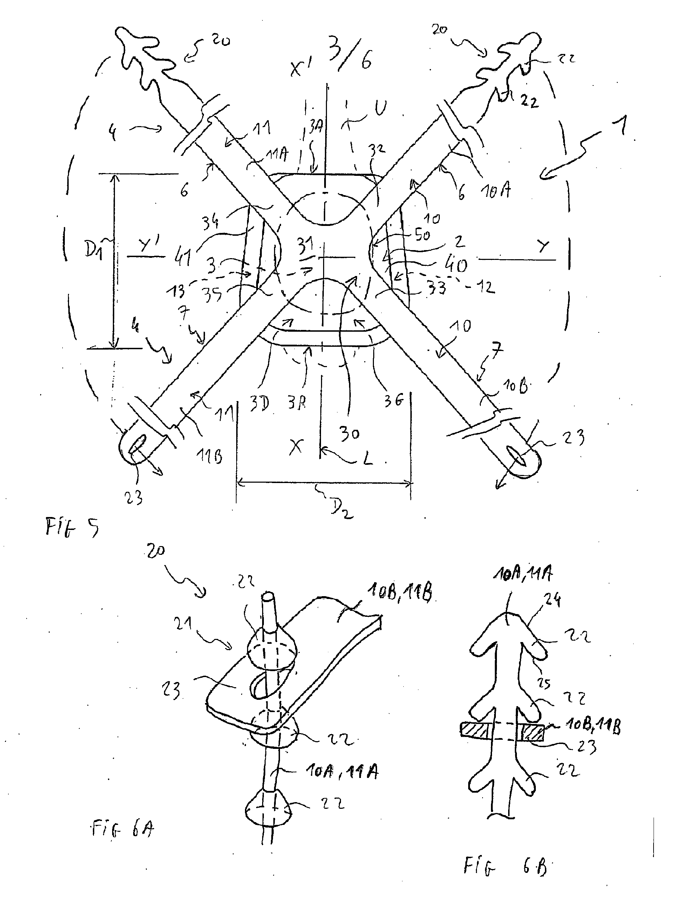

Plus particulièrement, tel que cela est représenté sur les figures 5, 6A et 6B, l'organe d'ajustement de longueur 21 peut comprendre une pluralité d'éléments de fixation « mâles » 22 répartis le long du premier brin 10A, 11A, ainsi qu'un ou plusieurs éléments de fixation « *femelles* » conjugués 23 associés au second brin 10B, 11B de telle sorte que la longueur effective de la bretelle 10, 11 correspondante puisse être ajustée, selon un pas d'incrément prédéterminé, en fonction du couple d'éléments de fixation 22, 23 choisi.

Tel que cela est illustré sur les figures 5, 6A et 6B, les éléments de fixation mâles 22 pourront notamment être formés par des excroissances coniques creuses ou pleines, ou encore des ancres comportant une ou plusieurs branches déformables, par exemple ménagées par découpe du brin constitutif de la bretelle.

Le ou les éléments de fixation femelles 23 pourront notamment être formée, par des orifices de passage de type boutonnière, aux lèvres éventuellement déformables, aptes à coopérer par encliquetage avec les éléments de fixation mâles 22.

A cet effet, les éléments de fixation mâles 22 associés au premier brin 10A, 11A présentent de préférence une face inclinée d'introduction 24 et une face de retenue opposée 25, le passage du premier brin à travers le second brin 10B, 11B s'effectuant en forçant la déformation des excroissances et/ou de l'orifice.

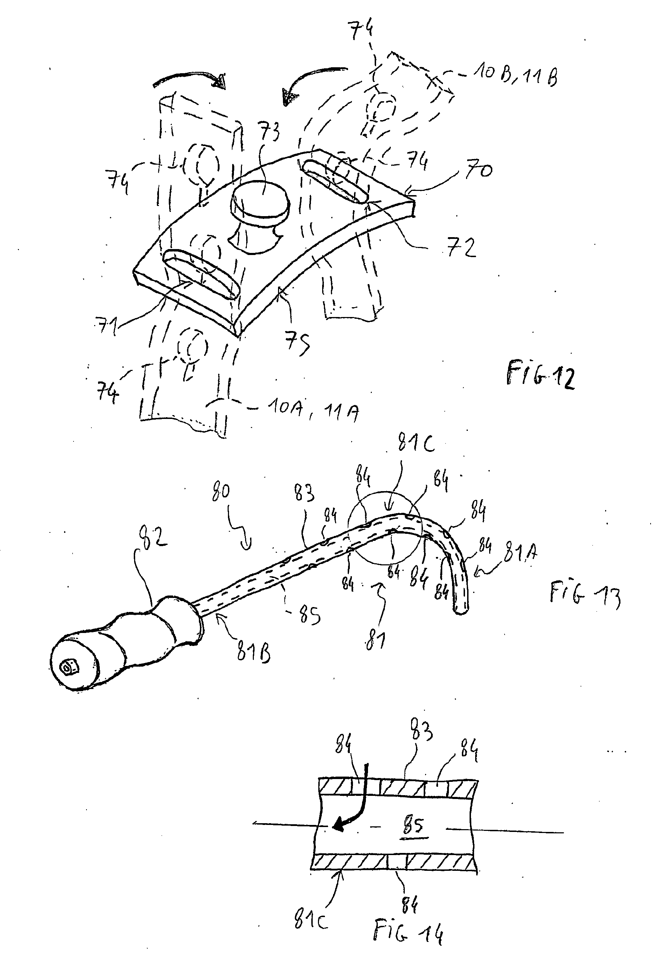

Selon une autre variante de réalisation illustrée sur la figure 12, on pourra employer un organe d'arrêt 70 percé dans son épaisseur d'un ou plusieurs orifices 71, 72 permettant le passage des brins (illustrés en pointillés sur la figure 12), et présentant une protubérance centrale 73 sur sa face supérieure, ladite protubérance formant un bouton d'amarrage avec lequel , coopèrent des boutonnières 74 percées dans les brins, lorsque lesdits brins sont rabattus contre l'organe d'arrêt 70 dans la direction indiquée par les flèches. Les boutonnières 74 peuvent notamment se présenter sous forme d'évidements circulaires fendus. '

Un tel agencement est avantageusement simple, peu onéreux et compact.

Eventuellement, l'organe d'arrêt 70 pourra comporter une embase 75 flexible et/ou incurvée destinée à venir reposer sur la partie supérieure de la symphyse pubienne et former une partie de l'organe porteur 5.

De préférence, le moyen de réglage 20 de la distance de soutènement sera conçu pour permettre une modification continue de la distance de soutènement d₀, c'est-à-dire autorisera sans restriction le libre choix de ladite distance de soutènement parmi l'ensemble des valeurs intermédiaires comprises dans une plage prédéterminée.

A cet effet, l'organe d'ajustement de longueur 21 comprend de préférence, tel que cela est illustré sur les figures 3, 7, 8A et 8B, une boucle de serrage 26 enfilée sur une suspente 6, 7 et agencée pour permettre un réglage continu de la longueur de ladite suspente.

Ladite boucle de serrage, qui pourra présenter certaines analogies avec les boucles utilisées sur les sangles de sacs à main ou de sacs à dos, comprendra par exemple un cadre 27, de préférence rigide ou semi-rigide, percé dans son épaisseur d'orifices 28 fixes ou encore pourvu de traverses coulissantes 29, ledit cadre étant destiné à accueillir les brins repliés sur eux-mêmes et à bloquer ces dernier par écrasement.

Ainsi, la boucle de serrage 26 jouera un rôle de fermoir permettant de réunir deux à deux les brins antérieur 10A et postérieur 10B d'une même bretelle 10 afin de reconstituer cette dernière en lui conférant la longueur voulue.

De préférence, la plage de réglage conférée par l'organe d'ajustement de longueur 21 conforme à l'invention, notamment dans le cas de moyens de réglage discrets, est sensiblement comprise entre 0,5 cm et 5 cm.

Il est remarquable que le moyen de réglage 20 de la distance de soutènement peut comporter à la fois des organes de réglage discret destinés à effectuer un réglage grossier de la longueur globale des bretelles en fonction de l'anatomie du patient, et des organes de réglage continu permettant un réglage fin de l'effort de suspension F.

Par ailleurs, selon une variante de réalisation préférentielle le moyen de soutènement 2 comprend un organe d'armature 30 relié aux suspentes 6, 7, tel que cela est illustré sur la figure 5.

Avantageusement, un tel organe d'armature 30 permet de renforcer le soutien sous-urétral et de déterminer un ou plusieurs points de compression particuliers.

Plus précisément, l'organe d'armature 30 est de préférence formé par un croisillon 31, dont le centre se trouve sensiblement à l'aplomb de la ligne moyenne (L) qui divise l'organe d'appui 3 en sa zone gauche et sa zone droite, et dont les branches 32, 33, 34, 35 sont reliées chacune à l'extrémité d'une bretelle 10, 11.

De façon particulièrement préférentielle, l'organe d'armature est venu de matière avec l'une et/ou l'autre des bretelles 10, 11.

Ainsi, tel que cela est illustré sur la figure 5, les branches 32, 33, 34, 35 du croisillon peuvent avantageusement former les extrémités captives des brins 10A, 10B, 11A, 11B, c'est-à-dire que le croisillon 31 est formé par la croisée sous-urétrale des bretelles gauche et droite 10, 11.

De préférence, l'organe d'appui 3 est formé, au moins en partie et de préférence en totalité, par une plaque souple 40.

Ladite plaque souple 40 peut par exemple être formée par une pièce de textile, notamment tricotée, ou de non tissé, à base d'un matériau colonisable tel que le PolyPropylène, afin d'assurer une intégration de ladite plaque souple 40 avec l'organe soutenu, en l'occurrence l'urètre U et/ou le muscle bulbo-caverneux, et de prévenir tout risque de glissement relatif dudit organe par rapport à ladite plaque souple 40.

Ladite plaque souple 40 est en outre de préférence apte à se déformer sensiblement en « U », tel que cela est illustré notamment sur les figures 3 et 4, pour envelopper partiellement l'urètre U et/ou, le cas échéant, l'ensemble formé par l'urètre U, le corps caverneux CC et le muscle bulbo-caverneux MBC.

La plaque souple 40 forme donc de préférence à la fois l'organe d'appui 3 et les organes de maintien latéral 12, 13.

Ainsi, le moyen de soutènement 2 peut avantageusement épouser les portions inférieures et latérales de l'urètre U et/ou du muscle bulbo-caverneux, de manière à répartir l'effort de suspension F sur une importante surface tissulaire d'une part, et à limiter le débattement de l'urètre U par rapport à la prothèse 1 et à la symphyse pubienne SP d'autre part

On obtient ainsi avantageusement une suspension particulièrement atraumatique et stable.

De préférence, l'organe d'armature 30 est flexible et solidaire de la plaque souple 40.

Ainsi, le moyen de soutènement peut présenter une structure multi-couches, dans laquelle les éléments d'armature 30 se trouvent à l'opposé de l'organe d'appui 3.

En particulier, le croisillon 31 peut être assujetti par collage ou suture à la plaque souple 40.

En outre, ledit croisillon 31 sera de préférence agencé de manière à ce que les bretelles émergent sensiblement aux coins du moyen de soutènement 2, et plus précisément de la plaque souple 40.

Il est remarquable que la présence des moyens d'armature 30 permet avantageusement d'une part de définir dans l'espace un point de compression préférentielle relativement rigide, sous l'organe d'appui 3, sensiblement à la croisée des bretelles, et d'autre part d'éviter le froncement de la plaque souple 40, ce qui facilite la mise en place de la prothèse et la tenue du moyen de soutènement 2 dans le temps.

Bien entendu, l'organe d'armature 30 n'est nullement limité à une géométrie particulière et pourrait notamment comporter une bordure de renfort suivant par exemple sensiblement les contours de la plaque souple 40.

Par ailleurs, la zone souple 40 comprend de préférence une ou plusieurs zones de suture 41 destinées à être suturées au muscle bulbo-carverneux MBC afin d'immobiliser ledit muscle bulbo-caverneux au sein du moyen de soutènement 2. De préférence, tel que cela est illustré sur la figure 5, la zone de suture 41 s'étendra sensiblement à la périphérie de la plaque souple 40.

Selon une variante de réalisation préférentielle, la profondeur D1 de l'organe d'appui 3, et plus globalement de la plaque souple 40, c'est-à-dire sa dimension (hors-tout) considérée selon la direction principale d'extension XX' de l'urètre U et « *à plat* », c'est-à-dire lorsque la prothèse 1 repose déployée sur un plan, est supérieure ou égale à sa largeur D2, c'est-à-dire à sa dimension (hors-tout) considérée selon une direction latérale transverse YY' à ladite direction principale d'extension XX' de l'urètre dans les mêmes conditions.

A titre indicatif, la profondeur D1 pourra être comprise sensiblement entre 40 mm et 60 mm, et la largeur D2 entre 30 mm et 50 mm.

Ainsi, le moyen de soutènement 2 forme avantageusement un hamac, ou une sorte de gouttière épousant la forme anatomique de l'urètre et/ou du muscle bulbo-caverneux et apte à répartir l'effort de suspension F sur une longueur significative.de celui-ci.

Par ailleurs, afin de s'adapter au mieux à l'anatomie du patient, l'organe d'appui 3, et plus globalement la plaque souple 40, est de préférence sensiblement quadrangulaire, et de façon particulièrement préférentielle sensiblement trapézoïdal, c'est-à-dire que la largeur de son bord antérieur 3A est sensiblement inférieure ou égale à la largeur de son bord postérieur 3P.

Par ailleurs, la prothèse 1 conforme à l'invention peut comporter un moyen de compression gonflable 50 qui est disposé au niveau de la face d'appui 3, entre le moyen de soutènement 2 et l'urètre U, et qui est agencé de manière à pouvoir, lorsqu'il est gonflé, venir exercer, tel que cela est représenté sur la figure 4, une compression de l'urètre contre les tissus qui surplombent ce dernier, par exemple le ligament transverse du pelvis.

Ainsi, avantageusement, la prothèse 1 conforme à l'invention peut combiner un effort de suspension F et un effort de compression P distincts, ledit effort de compression P étant capable de réduire la lumière urétrale afin d'exercer une fonction d'obturation suppléant une déficience sphinctérienne. La prothèse 1 conforme à l'invention peut donc constituer une alternative, avantageusement simple et peu onéreuse, aux sphincters artificiels de l'art antérieur.

Plus précisément, le moyen de compression gonflable 50 prend avantageusement appui sur la partie inférieure du moyen de soutènement 2 pour exercer une pression P complémentaire sur l'urètre qui tend à rapprocher la paroi inférieure de ce dernier de la symphyse pubienne.

De préférence, le moyen de compression gonflable 50 est formé par un coussin 51 qui est disposé sensiblement au droit de la ligne moyenne qui divise la surface de l'organe d'appui 3 sensiblement parallèlement à la direction principale d'extension XX' de l'urètre U, tel que cela est illustré sur la figure 4.

Ledit coussin 51 peut notamment être logée dans une poche *« kangourou* » ménagée sous la surface de l'organe d'appui 3 par un pli de la plaque souple 40.

Il est remarquable que cette variante de réalisation intègre de préférence un croisillon 31 tel que décrit ci-dessus de manière à fournir au coussin 51 un point d'appui particulièrement solide sur le moyen de soutènement 2. Ainsi, la contrainte de compression P agira de préférence de manière localisée et différentielle par rapport à l'effort global de suspension F.

De façon particulièrement préférentielle, le moyen de compression gonflable 50 est relié à un réservoir de fluide 54, tel qu'un site implantable, au moyen d'une tubulure souple de type cathéter 55.

De préférence, tel que cela est illustré sur la figure 3, le site implantable 54 est disposé en appui stable et rigide contre la face antérieure de la symphyse pubienne SP.

En outre, la prothèse 1 pourra comporter des moyens de transfert de fluide, tel qu'une poire ou une pompe télécommandée permettant au patient de commander à volonté le gonflage et le dégonflage du coussin 51 et ainsi d'autoriser la miction ou au contraire de garantir la continence.

Par ailleurs, la prothèse 1 conforme à l'invention peut avantageusement être conçue pour traiter une cervicocystoptose associée à l'incontinence chez la femme.

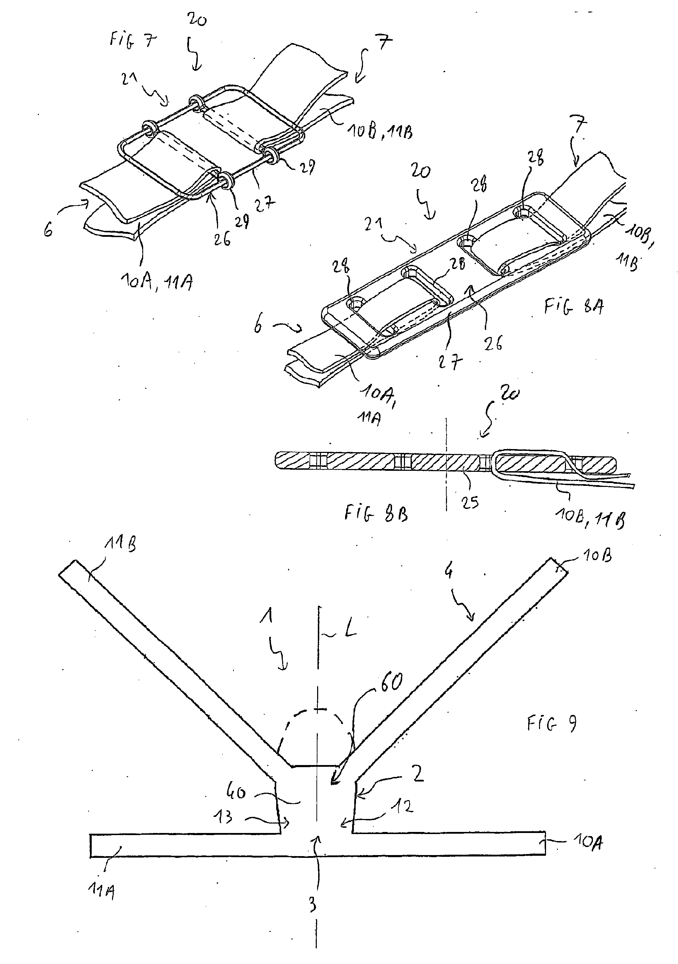

A cet effet, tel que cela est illustré sur les figures 9 et 11 le moyen de soutènement 2 peut présenter une extension sous-vésicale 60 conçue pour venir en appui sous la vessie V.

Par ailleurs, il est remarquable que, selon cette variante de réalisation, les brins postérieurs 10B, 11B sont orientés de préférence en oblique par rapport aux bords de la plaque souple, afin d'améliorer l'ergonomie de la prothèse 1.

Avantageusement, l'extension sous-vésicale 60 pourra s'étendre également au-delà des points d'attache des brins postérieurs au moyen de soutènement, entre lesdits brins postérieurs, et par exemple former une languette au contour arrondi, tel que cela est illustré en pointillés sur la figure 9.

Tel que cela est illustré sur la figure 11, l'extension sous-vésicale est conçue pour venir soutenir au moins la base de la vessie, tandis que les suspentes postérieures 7 sont agencées pour contourner latéralement la vessie V, de part et d'autre de cette dernière, et relier ladite extension sous-vésicale 60 à l'organe porteur 5.

Ainsi, la prothèse 1 peut venir étayer la vessie V en suspendant celle-ci, en même temps que l'urètre, à la symphyse pubienne.

Avantageusement, une seule intervention permet par conséquent de remédier simultanément à un prolapsus et à une incontinence.

Bien entendu, la présente invention n'est nullement limitée à une variante de réalisation particulière. Ainsi, la présente invention peut concerner en tant que telle, indépendamment de la présence de moyens de réglage de la distance de soutènement, une prothèse implantable destinée au traitement de l'incontinence qui comprend un moyen de soutènement présentant un organe d'appui conçu pour venir se placer sous l'urètre afin de faire obstacle à l'éloignement de ce dernier en direction de la partie inférieure du pelvis et d'empêcher tout écoulement intra-urétral incontrôlé, ladite prothèse comportant un moyen de suspension symphysaire conçu pour suspendre ledit moyen de soutènement à la symphyse pubienne et pour maintenir l'organe d'appui à une distance sensiblement constante de ladite symphyse pubienne, dite distance de soutènement.

Par ailleurs, il est décrit un ancillaire destiné à faciliter la mise en place de la prothèse 1 décrite ci-dessus, et plus particulièrement à permettre le passage des suspentes postérieures 7 dans l'espace rétro-pubien tout en garantissant que l'intégrité de la vessie est respectée.

Tel que cela est illustré sur les figures 13 et 14, l'ancillaire se présente sous la forme d'un instrument chirurgical 80 pourvu d'un organe d'introduction 81 qui est conçu pour être enfoncé dans les tissus situés dans l'espace rétro-pubien afin d'y ouvrir un passage destiné à l'une ou l'autre des suspentes 6, 7.

Ledit organe d'introduction 81 comprend au moins une portion proximale 81A agencée pour pénétrer dans les tissus, une portion distale 81 B rattachée à un moyen de préhension 82, de type poignée, et un tronçon intermédiaire 81C qui relie la portion proximale 81A à la portion distale 81 B et présente une surface latérale extérieure 83 au contact des tissus traversés.

Le tronçon intermédiaire 81C comporte au moins un orifice latéral 84 reliant sa surface latérale extérieure 83 à un canal de collecte 85 de sorte à pouvoir recueillir le liquide contenu dans la vessie lorsque l'organe d'introduction perfore accidentellement cette dernière.

Ainsi, un écoulement de liquide urinaire ou de liquide coloré préalablement injecté dans la vessie informe immédiatement le chirurgien que l'organe d'introduction 81 traverse la vessie et draine son contenu, c'est-à-dire que ladite vessie a été perforée accidentellement et qu'il est par conséquent nécessaire d'intervenir pour restaurer son fonctionnement.

Bien entendu, l'organe d'introduction 81 présente une rigidité suffisante pour lui permettre de percer un passage dans les tissus et de cheminer à travers ces derniers lorsque le chirurgien exerce une poussée sur l'organe de préhension 82.

A cet effet, l'extrémité de la portion proximale 81C peut être pourvue d'un organe de pénétration, par exemple d'une pointe, éventuellement émoussée et/ou amovible, de préférence en matériau polymère.

En outre, l'organe d'introduction sera de préférence incurvé pour former une sorte de crochet et ainsi faciliter l'aménagement d'un passage péri-symphysaire, sans risque de perforer des régions anatomiques plus profondes.

En outre, l'organe d'introduction 81 présentera de préférence une longueur suffisante pour que sa portion proximale 81A puisse ressurgir hors des tissus, sous le pubis, tandis que sa portion distale 81B comprenant le moyen de préhension 82 émerge encore des tissus sus-pubiens.

L'organe d'introduction peut être formé à partir d'une tige recourbée et creuse, par exemple métallique, présentant de préférence une géométrie cylindrique de base circulaire, le canal de collecte 85 courant à l'intériéur de ladite tige pour déboucher à l'air libre ou dans une poche prévue à cet effet au niveau de l'extrémité distale 81B.

De préférence, l'instrument 80 comporte une pluralité d'orifices latéraux 84 percés sensiblement radialement dans l'épaisseur de la paroi de la tige et étagés le long de l'organe d'introduction.

Selon une variante, les orifices latéraux 84 sont disposés en plusieurs points de la circonférence de l'organe d'introduction, de sorte à pouvoir drainer le liquide vésical quelle que soit l'orientation dudit organe d'introduction 81.

Ledit organe d'introduction 81 est donc de préférence multi-perforé, tant sur sa longueur que sur sa périphérie.

Bien que l'ancillaire soit particulièrement adapté à la mise en oeuvre d'une prothèse 1 telle que décrite plus haut, son usage n'y est nullement limité. Ainsi, il est parfaitement envisageable d'adapter à d'autres usages un instrument chirurgical comportant un organe d'introduction pourvu d'un ou plusieurs orifices latéraux 84 reliant sa surface latérale extérieure 83 à un canal de collecte 85 de sorte à pouvoir recueillir le liquide contenu lorsque ledit organe d'introduction perfore accidentellement un organe.

Une méthode va maintenant être brièvement décrite, en référence à la mise en place d'une prothèse 1 conforme à l'invention à l'aide d'un instrument chirurgical 80.

Plus particulièrement, la méthode décrite fait référence à une suspension péri-symphysaire, étant entendu qu'une méthode sensiblement analogue peut être appliquée pour obtenir une suspension péri-osseuse aux branches ischio-pubiennes ou ilio-pubiennes par la voie transobturatrice.

Ladite méthode chirurgicale comprend une étape (a) d'engagement au cours de laquelle on engage sous l'urètre U un moyen de soutènement 2 comprenant un organe d'appui 3 conçu pour faire obstacle à l'éloignement de l'urètre en direction de la partie inférieure du pelvis et empêcher tout écoulement intra-urétral incontrôlé.

Chez l'homme, ledit moyen de soutènement 2 sera de préférence disposé sous le muscle bulbo-caverneux MBC, au contact de celui-ci, tandis que chez la femme, l'organe d'appui 3 du moyen de soutènement 2 viendra de préférence au contact de l'urètre U lui-même.

Ladite méthode de chirurgicale comprend également une étape (b) de suspension osseuse atraumatique au cours de laquelle on suspend le moyen de soutènement à l'os pubien à l'aide d'un moyen de suspension atraumatique 4 de telle sorte que l'organe d'appui 3 soit maintenu à une distance sensiblement constante de l'os pubien OP, et plus particulièrement de la symphyse pubienne SP, dite distance de soutènement d₀.

De préférence, l'étape (b) de suspension constitue une étape de suspension à la symphyse pubienne SP.

Ainsi, on *« emprisonne* », ou on enserre, l'urètre U dans une région de l'espace délimitée par la symphyse pubienne SP d'une part et l'organe d'appui 3 situé à une distance d₀ d'autre part.

De façon particulièrement préférentielle, l'étape (b) de suspension atraumatique comprend une sous-étape (b1) de franchissement péri-osseux, et plus particulièrement péri-symphysaire, au cours de laquelle on dispose un organe porteur 5 à cheval une portion de l'os pubien, et plus particulièrement sur la symphyse pubienne SP, et l'on relie le moyen de soutènement 2 audit organe porteur 5 à l'aide d'au moins une suspente antérieure 6 passant devant l'os pubien, respectivement la symphyse pubienne SP, et d'au moins une suspente postérieure 7 passant derrière ledit os pubien, respectivement ladite symphyse pubienne SP, dans l'espace rétro-pubien.

L'accès à la région anatomique concernée dépend notamment du sexe du patient.

Chez l'homme, le chirurgien réalise de préférence une incision péno-scrotale horizontale, transversale, qui permet, après incision longitudinale des plans superficiels (tissu cellulaire sous-cutané et aponévrose périnéale superficielle), d'exposer les faces inférieures et latérales de l'urètre et du muscle bulbo-caverneux.

Le chirurgien introduit ensuite son index dans l'angle situé entre le muscle bulbo-urétral en dedans et la branche du corps caverneux en dehors, de manière à repérer le bord inférieur de la branche ischio-pubienne et d'amorcer le passage (ascendant).

Le chirurgien pratique ensuite une incision sus-pubienne transversale de 2 à 3 cm, à deux travers de doigts de chaque côté de la ligne médiane jusqu'à la gaine du muscle du grand droit, qui est également incisée. Le chirurgien introduit alors son second index dans l'incision pour créer de haut en bas, un passage au ras de la face postérieure du pubis jusqu'au plancher pelvien et venir opérer la jonction avec l'index déjà introduit de bas en haut par l'incision péno-scrotale.

Un ancillaire adapté passé de haut en bas à travers le passage ainsi formé permet de saisir le brin postérieur et de le tirer jusqu'à l'extérioriser par l'incision sus-pubienne.

Avantageusement, le chirurgien peut employer un instrument chirurgical 80 pour créer le passage (opération de « *forage d'un tunnel* ») puis pour saisir le brin postérieur.

A cet effet, il introduit à force l'organe d'introduction 81 par l'incision sus-pubienne et l'enfonce en faisant progressivement basculer sa partie recourbée autour de la symphyse pubienne, jusqu'à ce que l'extrémité proximale ressurgisse à l'air libre.

De préférence, l'étape de forage au moyen de l'instrument est précédée d'une étape de remplissage de la vessie par un liquide coloré pour faciliter la détection d'une éventuelle complication.

En cas d'écoulement anormal par le canal de collecte 85 au cours de cette opération, le praticien procède à une fibroscopie de contrôle pour diagnostiquer la nature et l'étendue de la perforation vésicale en vue d'y remédier.

La même manoeuvre est répétée du côté opposé (à droite si le premier geste a été effectué à gauche) pour mettre en place le second brin postérieur.

Un ancillaire de saisie est ensuite introduit par la même incision sus-pubienne puis passé en avant du pubis pour rejoindre l'espace latéro-urétral et saisir le brin antérieur pour hisser ce dernier jusqu'à l'extérioriser par ladite incision sus-pubienne.

Chez la femme, l'exposition de l'urètre est obtenue de préférence au moyen d'une incision verticale de la paroi vaginale antérieure. Le geste chirurgical permettant le passage des brins constitutifs des bretelles est réalisé de manière sensiblement analogue à ce qui a été décrit pour l'homme.

La méthode chirurgicale comprend également une étape (c) de réglage de tension de suspension au cours de laquelle le praticien ajuste *in vivo* la distance de soutènement d₀ afin de régler l'effort de suspension F exercé par l'organe d'appui 3 sur l'urètre U.

Plus précisément, ladite étape (c) de réglage de tension de suspension peut comporter une sous-étape (c1) d'ajustement de la longueur des suspentes 6, 7 au cours de laquelle on règle la longueur de l'une ou l'autre des suspentes 6, 7, et plus précisément des bretelles 10, 11.

A cet effet, le chirurgien peut joindre le brin antérieur 10A et le brin postérieur 10B correspondant au moyen d'une boucle de serrage 26 afin de reconstituer la bretelle correspondante 10, puis ajuster la longueur de ladite bretelle *« coulante »* en tirant sur l'extrémité libre de l'un ou l'autre brin 10A, 10B passée dans ladite boucle de serrage 26.

En particulier, le chirurgien peut sélectionner une longueur initiale de bretelle puis procéder itérativemént, par une succession d'opération d'allongement et/ou de raccourcissement, à la sélection de la longueur la plus appropriée en vue de l'effort de suspension à obtenir.

Chez l'homme, la méthode chirurgicale peut également comprendre une étape (d) de stabilisation au cours de laquelle on suture le muscle bulbo-caverneux MBC au moyen de soutènement 2, et plus précisément à la plaque souple 40. Ladite étape (d) de stabilisation intervient de préférence avant l'étape (c) de réglage de la tension de suspension, et plus précisément, avant la reconstitution des bretelles par assemblage de leurs brins respectifs.

Enfin, chez la femme, la méthode chirurgicale peut comporter une étape (e) de suspension vésicale au cours de laquelle on introduit une extension sous-vésicale 60 du moyen de soutènement 2 sous la vessie V afin de soutenir conjointement l'urètre et ladite vessie.

Plus particulièrement, ladite étape (e) de suspension sous-vésicale peut comprendre une sous-étape (e1) au cours de laquelle on relie, au moyen d'une suspente postérieure 7, l'extrémité proximale de l'extension sous-vésicale 60 à l'organe porteur 5, en faisant passer les deux brins postérieurs 10B, 11B latéralement, de part et d'autre de la vessie.

Bien entendu, la mise en place d'une telle extension sous-vésicale requiert une dissection sous-vésicale plus poussée que celle décrite précédemment. Le réglage de la tension de suspension F reste avantageusement identique à celui décrit plus haut.

Ainsi, la prothèse conforme à l'invention permet avantageusement de traiter efficacement la quasi-totalité des cas d'incontinence urinaire pour un coût modéré et sans occasionner de traumatisme important pour le patient.

En outre, l'action différentielle exercée d'une part par les moyen de suspension et d'autre part les moyens de compression gonflables permet d'adapter aisément ladite prothèse à la sévérité de l'incontinence à traiter.

Enfin, la stabilité et le caractère atraumatique de ladite prothèse 1 réduisent considérablement la probabilité de devoir pratiquer une ré-intervention en minimisant l'érosion tissulaire, tant au niveau de l'urètre que des points d'appui permettant la suspension de la prothèse.

### POSSIBILITE D'APPLICATION INDUSTRIELLE

L'invention trouve son application industrielle dans la conception et la fabrication de prothèses de traitement de l'incontinence.

## Revendications

1. Prothèse (1) implantable destinée au traitement de l'incontinence comprenant un moyen de soutènement (2) présentant un organe d'appui (3) conçu pour venir se placer sous l'urètre (U) afin de faire obstacle à l'éloignement de ce dernier en direction de la partie inférieure du pelvis et d'empêcher tout écoulement intra-urétral incontrôlé, ladite prothèse comportant :
- un moyen de suspension atraumatique (4) pourvu d' au moins deux bretelles de suspension (10, 11) comprenant chacune un organe porteur (5) agencé pour chevaucher une partie de l'os pubien (OP) et prendre appui sur ce dernier, ainsi qu'au moins une suspente antérieure (6) et une suspente postérieure (7) agencées pour relier l'organe porteur (5) au moyen de soutènement (2) en passant respectivement devant et derrière l'os pubien (OP), lesdites bretelles de suspension (10, 11) étant conçue pour maintenir l'organe d'appui (3) à une distance sensiblement constante de l'os pubien (OP), dite distance de soutènement (do)
- un moyen de réglage réversible (20) de la distance de soutènement (do) permettant de faire varier de manière réversible ladite distance de soutènement (do).

2. Prothèse selon la revendication 1 **caractérisée en ce que** le moyen de réglage réversible (20) de la distance de soutènement (do) possède un organe d'ajustement de longueur (21) de l'une ou l'autre des suspentes (6, 7).

3. Prothèse selon l'une des revendications précédentes **caractérisée en ce que** le moyen de soutènement (2) présente une zone gauche (3G) et une zone droite (3D) situées de part et d'autre d'une ligne moyenne (L) fictive sensiblement parallèle à la direction principale d'extension (XX') de l'urètre, et **en ce que** le moyen de suspension (4) possède une bretelle gauche (10) et une bretelle droite (11), lesdites bretelles gauche et droite étant respectivement fixées au moyen de soutènement (2) dans sa zone gauche et dans sa zone droite.

4. Prothèse selon l'une des revendications précédentes **caractérisée en ce que** le moyen de soutènement (2) comprend un organe d'armature (30) relié aux suspentes (6, 7).

5. Prothèse selon la revendication 4 et l'une des revendications 1 à 3 **caractérisée en ce que** l'organe d'armature (30) est formé par un croisillon (31), dont le centre se trouve sensiblement à l'aplomb de la ligne moyenne (L) et dont les branches (32, 33, 34, 35) sont reliées chacune à l'extrémité d'une bretelle (10, 11).

6. Prothèse selon la revendication 4 ou 5 **caractérisée en ce que** l'organe d'armature (30) est venu de matière avec !'une et/ou l'autre des suspentes (6, 7).

7. Prothèse selon l'une des revendications 4 à 6 **caractérisée en ce que** l'organe d'appui (3) est formé, au moins en partie et de préférence en totalité, par une plaque souple (40) et **en ce que** l'organe d'armature (30) est flexible et solidaire de ladite plaque souple (40).

8. Prothèse selon l'une des revendications précédentes **caractérisée en ce que** le moyen de soutènement (2) comporte des organes de maintien latéral (12, 13) conçus pour venir se positionner de chaque côté de l'urètre.

9. Prothèse selon l'une des revendications précédentes **caractérisée en ce que** l'organe d'appui (3) est formé, au moins en partie et de préférence en totalité, par une plaque souple (40) et **en ce que** la profondeur (D1) de ladite plaque souple (40) considérée selon la direction principale d'extension (XX') de l'urètre est supérieure ou égale à sa largeur (D2) considérée selon une direction latérale transverse (YY') à ladite direction principale d'extension (XX') de l'urètre.

10. Prothèse selon l'une des revendications précédentes **caractérisée en ce que** l'organe d'appui (3) est formé, au moins en partie et de préférence en totalité, par une plaque souple (40) dont la largeur du bord antérieur est sensiblement inférieure ou égale la largeur de son bord postérieur.

11. Prothèse selon l'une des revendications précédentes **caractérisée en ce que** le moyen de suspension (4) constitue un moyen de suspension péri-symphysaire conçu pour suspendre le moyen de soutènement (2) à la symphyse pubienne (SP).

12. Prothèse selon l'une des revendications précédentes **caractérisée en ce qu'**elle comporte un moyen de compression gonflable (50) qui est disposé au niveau de l'organe d'appui (3), entre le moyen de soutènement (2) et l'urètre (U), et agencé de manière à pouvoir, lorsqu'il est gonflé, exercer une compression de l'urètre contre les tissus qui surplombent ce dernier.

13. Prothèse selon l'une des revendications précédentes **caractérisée en ce que** le moyen de soutènement (2) présente une extension sous- vésicale (60) conçue pour venir en appui sous la vessie.

## Patentansprüche

1. Implantierbare Prothese (1), die zur Behandlung der Inkontinenz bestimmt ist, mit einer Stützeinrichtung (2), die ein Auflageelement (3) aufweist, das konzipiert ist, um sich unter der Harnröhre (U) zu platzieren, um ein Hindernis für deren Entfernung in Richtung des unteren Bereichs des Beckens zu bilden und jedes unkontrollierte intraurethrale Fließen zu verhindern, wobei die Prothese aufweist:
- eine atraumatische Aufhängeeinrichtung (4), die mit mindestens zwei Aufhänge-Tragriemen (10, 11) versehen ist, die je ein Trägerelement (5), das eingerichtet ist, um einen Bereich des Schambeins (OP) zu überlappen und auf diesem aufzuliegen, sowie mindestens einen vorderen Tragegurt (6) und einen hinteren Tragegurt (7) enthalten, die eingerichtet sind, um das Trägerelement (5) mit der Stützeinrichtung (2) zu verbinden, indem sie vor bzw. hinter dem Schambein (OP) verlaufen, wobei die Aufhänge-Tragriemen (10, 11) konzipiert sind, um das Auflageelement (3) in einem im Wesentlichen konstanten Abstand vom Schambein (OP), Stützabstand (do) genannt, zu halten,
- eine Einrichtung (20) zur reversiblen Regelung des Stützabstands (do), die es ermöglicht, den Stützabstand (do) reversibel zu variieren.

2. Prothese nach Anspruch 1, **dadurch gekennzeichnet, dass** die Einrichtung (20) zur reversiblen Regelung des Stützabstands (do) ein Längeneinstellelement (21) des einen oder anderen der Tragegurte (6, 7) besitzt.

3. Prothese nach einem der vorhergehenden Ansprüche, **dadurch gekennzeichnet, dass** die Stützeinrichtung (2) eine linke Zone (3G) und eine rechte Zone (3D) aufweist, die sich zu beiden Seiten einer fiktiven Mittellinie (L) im Wesentlichen parallel zur Hauptausdehnungsrichtung (XX') der Harnröhre befinden, und dass die Aufhängeeinrichtung (4) einen linken Tragriemen (10) und einen rechten Tragriemen (11) besitzt, wobei der linke und der rechte Tragriemen an der Stützeinrichtung (2) in ihrer linken bzw. rechten Zone befestigt sind.

4. Prothese nach einem der vorhergehenden Ansprüche, **dadurch gekennzeichnet, dass** die Stützeinrichtung (2) ein Armierungselement (30) enthält, das mit den Tragegurten (6, 7) verbunden ist.

5. Prothese nach Anspruch 4 und einem der Ansprüche 1 bis 3, **dadurch gekennzeichnet, dass** das Armierungselement (30) von einer Kreuzverstrebung (31) gebildet wird, deren Mitte sich im Wesentlichen senkrecht zur Mittellinie (L) befindet und deren Schenkel (32, 33, 34, 35) je mit dem Ende eines Tragriemens (10, 11) verbunden sind.

6. Prothese nach Anspruch 4 oder 5, **dadurch gekennzeichnet, dass** das Armierungselement (30) aus einem Stück mit dem einen und/oder anderen der Tragegurte (6, 7) hergestellt wird.

7. Prothese nach einem der Ansprüche 4 bis 6, **dadurch gekennzeichnet, dass** das Auflageelement (3) zumindest zum Teil und vorzugsweise ganz von einer biegsamen Platte (40) geformt wird, und dass das Armierungselement (30) elastisch und fest mit der biegsamen Platte (40) verbunden ist.

8. Prothese nach einem der vorhergehenden Ansprüche, **dadurch gekennzeichnet, dass** die Stützeinrichtung (2) seitliche Halteelemente (12, 13) aufweist, die konzipiert sind, um sich zu beiden Seiten der Harnröhre zu positionieren.

9. Prothese nach einem der vorhergehenden Ansprüche, **dadurch gekennzeichnet, dass** das Auflageelement (3) zumindest zum Teil und vorzugsweise ganz von einer biegsamen Platte (40) gebildet wird, und dass die Tiefe (D1) der biegsamen Platte (40) gesehen in der Hauptausdehnungsrichtung (XX') der Harnröhre größer als die oder gleich ihrer Breite (D2) gesehen in einer seitlichen Querrichtung (YY') zur Hauptausdehnungsrichtung (XX') der Harnröhre ist.

10. Prothese nach einem der vorhergehenden Ansprüche, **dadurch gekennzeichnet, dass** das Auflageelement (3) zumindest zum Teil und vorzugsweise ganz von einer biegsamen Platte (40) gebildet wird, deren Breite des vorderen Rands im Wesentlichen kleiner als die oder gleich der Breite ihres hinteren Rands ist.

11. Prothese nach einem der vorhergehenden Ansprüche, **dadurch gekennzeichnet, dass** die Aufhängeeinrichtung (4) eine außerhalb der Schambeinfuge befindliche Aufhängeeinrichtung bildet, die konzipiert ist, um die Stützeinrichtung (2) an der Schambeinfuge (SP) aufzuhängen.

12. Prothese nach einem der vorhergehenden Ansprüche, **dadurch gekennzeichnet, dass** sie eine aufblasbare Kompressionseinrichtung (50) aufweist, die im Bereich des Auflageelements (3) zwischen der Stützeinrichtung (2) und der Harnröhre (U) angeordnet und eingerichtet ist, um, wenn sie aufgeblasen ist, eine Kompression der Harnröhre gegen die diese überragenden Gewebe auszuüben.

13. Prothese nach einem der vorhergehenden Ansprüche, **dadurch gekennzeichnet, dass** die Stützeinrichtung (2) eine Ausdehnung (60) unter der Blase aufweist, die konzipiert ist, um unter der Blase in Auflage zu kommen.

## Claims

1. Implantable prosthesis (1) for the treatment of incontinence, comprising a retaining means (2) with a supporting member (3) designed to be placed under the urethra (U) in order to form an obstacle to the latter moving away in the direction of the lower pelvis and to prevent any uncontrolled intra-urethral flow, said prosthesis having:
- an atraumatic suspension means (4) provided with at least two suspension harnesses (10, 11), each comprising a carrying member (5) designed to straddle part of the pubic bone (OP) and to bear on the latter, and at least one anterior sling (6) and posterior sling (7) designed to connect the carrying member (5) to the retaining means (2) by passing respectively in front of and behind the pubic bone (OP), said suspension harnesses (10, 11) being designed to keep the supporting member (3) at a substantially constant distance from the pubic bone (OP), called the retaining distance (do),
- a means (20) for reversibly adjusting the retaining distance (do), thus making it possible to reversibly vary said retaining distance (do).

2. Prosthesis according to Claim 1, **characterized in that** the means (20) for reversibly adjusting the retaining distance (do) has a member (21) for adjusting the length of one or the other of the slings (6, 7).

3. Prosthesis according to one of the preceding claims, **characterized in that** the retaining means (2) has a left area (3G) and a right area (3D) situated to both sides of an imaginary centre line (L) substantially parallel to the main extension direction (XX') of the urethra, and **in that** the suspension means (4) has a left harness (10) and a right harness (11), said left and right harnesses being fixed to the retaining means (2) respectively in the left area and in the right area of the latter.

4. Prosthesis according to one of the preceding claims, **characterized in that** the retaining means (2) comprises a reinforcing member (30) connected to the slings (6, 7).

5. Prosthesis according to Claim 4 and one of Claims 1 to 3, **characterized in that** the reinforcing member (30) is formed by a crosspiece (31) whose centre is located substantially in alignment with the centre line (L) and whose branches (32, 33, 34, 35) are each connected to the end of a harness (10, 11).

6. Prosthesis according to Claim 4 or 5, **characterized in that** the reinforcing member (30) is made in one piece with one and/or the other of the slings (6, 7).

7. Prosthesis according to one of Claims 4 to 6, **characterized in that** the supporting member (3) is formed at least in part, and preferably completely, by a flexible plate (40), and **in that** the reinforcing member (30) is flexible and integrally connected to said flexible plate (40).

8. Prosthesis according to one of the preceding claims, **characterized in that** the retaining means (2) has lateral holding members (12, 13) designed to be positioned on each side of the urethra.

9. Prosthesis according to one of the preceding claims, **characterized in that** the supporting member (3) is formed at least in part, and preferably completely, by a flexible plate (40), and **in that** the depth (D1) of said flexible plate (40), considered along the main extension direction (XX') of the urethra, is greater than or equal to its width (D2), considered along a lateral direction (YY') transverse to said main extension direction (XX') of the urethra.

10. Prosthesis according to one of the preceding claims, **characterized in that** the supporting member (3) is formed at least in part, and preferably completely, by a flexible plate (40) in which the width of the anterior edge is substantially less than or equal to the width of its posterior edge.

11. Prosthesis according to one of the preceding claims, **characterized in that** the suspension means (4) constitutes a peri-symphyseal suspension means designed to suspend the retaining means (2) from the pubic symphysis (SP).

12. Prosthesis according to one of the preceding claims, **characterized in that** it has an inflatable compression means (50) which is arranged at the area of the supporting member (3), between the retaining means (2) and the urethra (U), and which is designed in such a way that, when inflated, it is able to exert a compression of the urethra against the tissues overhanging the latter.

13. Prosthesis according to one of the preceding claims, **characterized in that** the retaining means (2) has a subvesical extension (60) designed to bear from below on the bladder.
